# EUROPEAN PATENT APPLICATION

(11) **EP 3 511 783 A1**
(43) Date of publication of application: **17.07.2019**
(21) Application number: 18190954.0
(22) Date of filing: 27.08.2018
(51) Int. Cl.: G05B 17/00

(54) **METHOD FOR IMPROVING A CHEMICAL PRODUCTION PROCESS**

(30) Priority: 15.01.2018 EP 18151728
(71) Applicant: Covestro Deutschland AG, 51373 Leverkusen (DE)
(72) Inventor: The designation of the inventor has not yet been filed
(74) Representative: Levpat

(57) **Abstract**

The invention relates to a method for improving a chemical production process, wherein a plurality of derivative chemical products (1a, b) are produced through a derivative chemical production process based on at least some derivative process parameters (3a, b) at a respective chemical production facility (2a, b), which chemical production facilities (2a, b) each comprises a separate respective facility intranet (4a, b), wherein at least some respective derivative process parameters (3a, b) are measured from the derivative chemical production process by a respective production sensor computer system (5a, b) within each facility intranet (4a, b), wherein a process model (6) for simulating the derivative chemical production process is recorded in a process model management computer system (7) outside the facility intranets (4a, b). The method is characterized in that, that the process model (6) is transmitted to respective computing modules (8a, b) for performing numerical analysis within each facility intranet (4a, b) from the process model management computer system (7), that the derivative process parameters (3a, b) at each chemical production facility (2a, b) are provided to the respective computing module (8a, b) and that each computing module (8a, b) determines respective modification data (9a, b) for updating the process model (6) by a numerical analysis based on the derivative process parameters (3a, b) at the respective chemical production facility (2a, b) and the process model (6). The invention also relates to a corresponding system for a chemical production process.

## Description

The invention is directed at a method for improving a chemical production process with the features of the preamble of claim 1 and a system for a chemical production process with the features of the preamble of claim 15.

Chemical production processes are complex. The results are dependent on a very large number of variables and parameters. It is regularly the case that not only a large portion of these variables and parameters is not measured, either because their relevance is not recognized or measurement would be too difficult, but also that their effect on the product is not known. Sometimes there is no theoretical groundwork for stipulating such a dependency. Sometimes even when a dependency is suspected, there is insufficient data to determine such a dependency with sufficient accuracy. Yet recognizing and acting on such dependencies would be an important step in coming closer to desired properties of the final product and in reducing the proportion of defective products. This aspect becomes even more important when a chemical product is produced in separate steps which may also be undertaken at different facilities which are distant from each other. For example, parameters in the production process of a precursor may be relevant for properties of a final product made from the precursor in a chemical production process.

Advances in sensor and especially in computing technology have made it possible to not only accumulate very large amounts of data, but also to numerically process those very large amounts of data within reasonable time and at reasonable costs. By applying computational methods such as regression analysis on these large amounts of data, which regression analysis may be considered to be a form of data mining, it becomes possible to recognize and model properties of the chemical production process which where hitherto unknown. Thus, even if no theoretical basis for dependencies between process parameters and properties of the final product exists, such dependencies may be discovered by brute-force "number crunching". Even when a theoretical basis does exist, its predictive power may be vastly improved. Such dependencies may then be used for arriving at a construct modeling the process, which may also be called a process model. Further computational analysis with additional data and in light of the model may then result in iterative extensions, corrections or other amendments to the process model. Such a model may then be exploited for improving the process on the one hand and may also provide the starting point for additional research in order to arrive at a theoretical concept underlying the observed phenomenon.

Such a method for improving a chemical production process is known in particular from EP 1 364 262 B1. According to the teaching from this prior art, data from various sources is combined, shared and subjected to numerical analysis in order to determine and continually improve a process model for the chemical production process at hand.

For such an approach, the best results can be achieved when the sample size, i.e. the amount of data taken, as well as the number of sites and the number of production runs for the data is as large as possible. Thus, it is advantageous to combine as much data as possible from as many sources as possible. However, there are problems associated with such unrestricted sharing and combining of data. Firstly, for a chemical production facility both process parameters and production results, in particular the proportion of production deficiencies, present highly sensitive data, the dissemination of which to competitors would be hugely detrimental from a business perspective. Even with respect to a supplier of precursor material or some "neutral" party just engaged in data processing, sharing of such information would hinge on consent on the part of the chemical production facility. But even if the third-party recipient then did everything in his power to keep that data confidential, the mere fact of its sharing would at least increase the vulnerability of that data with respect to cybercrime. Likewise, also the party owning the process model will have an interest in keeping at least some of its information confidential, especially when it is a supplier, since otherwise its customer will see an improvement in their negotiation position when renewing contracts and when there are alternative suppliers available. Therefore, unrestrained information sharing may be advantageous from the point of view of numerical analysis, but is hampered by a number of different concerns which are, however, regularly assigned at least equal and often more importance.

Proceeding from the closest prior art, therefore the object of the invention is to provide a method for improving a chemical production process which allows exploiting large amounts of data from different sources while also ensuring that problems related to sharing of data are reduced.

With respect to a method for improving a chemical production process with the features of the preamble of claim 1, the object of the invention is achieved through the features of the characterizing part of claim 1. With respect to a system for a chemical production process with the features of the preamble of claim 15, the object of the invention is achieved through the features of the characterizing part of claim 15.

The invention is based on the recognition that an actual combining of data from different "owners" is only required at specific points and for specific purposes and that as a result of that combining, further data may be generated that in turn can be shared more safely. Thus, the data from a plurality of facilities from different, potentially competing parties may be used to improve a combined process model underlying the chemical production process at all these facilities without undue dissemination of sensitive information, if such combining occurs separately and locally at each of these facilities. The result of such combining, being useful for improving the process model, may then be transmitted to a central entity "owning" that process model such that no sensitive data is transmitted with it. Thereby, data from all the facilities may be put to use in a manner retaining the advantages of pooling but without the disadvantage of information dissemination.

The method according to the invention is for improving a chemical production process, wherein a plurality of derivative chemicals product are produced through a derivative chemical production process based on at least some derivative process parameters at a respective chemical production facility. Thus, there are a number of derivative process parameters, on at least some of which the derivative chemical production process is based. Any production process involving a chemical reaction may be understood to present a chemical production process. The expression "derivative chemical product" only signifies that there is at least one precursor material used in the derivative chemical production process, which shall be described in more detail below. A "chemical reaction" shall comprise in a traditional sense any chemical transformation of at least one precursor material to another chemical product, i.e. a derivative chemical product, as well as mixing of different precursor materials thus producing a mixture as a derivative chemical product.

In the method according to the invention, the chemical production facilities each comprises a separate respective facility intranet, wherein at least some respective derivative process parameters are measured from the derivative chemical production process by a respective production sensor computer system within each facility intranet and wherein a process model for simulating the derivative chemical production process is recorded in a process model management computer system outside each facility intranet.

The method of the invention is characterized in that the process model is transmitted to respective computing modules for performing numerical analysis within each facility intranet from the process model management computer system, that the derivative process parameters at each chemical production facility are provided to the respective computing module and that each computing module determines respective modification data for updating the process model by a numerical analysis based on the derivative process parameters at the respective chemical production facility and the process model. It is to be noted that the above transmission from the process model management computer system to the computing module does not necessarily involve a sending from the process model management computer system from its own initiative. Instead, such transmission may wholly or partially rely on the receiving activity of the computing module. In particular, the computing module may fetch or retrieve the process model from the process model management computer system.

The expression "within each facility intranet" means that the entity in question is communicatively coupled to the respective facility intranet such that it is to be considered inside the respective facility intranet and therefore enjoys the appropriate privileges for communication within that facility intranet. Conversely, the expression "outside the facility intranets" means that the entity in question may in principle be able to communicate with a computer within any of the facility intranets, but that it is not privileged in the same way as a computer within any of the facility intranets. Each computing module may consist of dedicated computing hardware, such as a personal computer or embedded computer, with appropriate software running on that computing hardware. Each computing module may consist of software only, running as a module on some computing hardware, such as a server, with different software unrelated to and separate from the computing module also running on the same computing hardware. It may also be that the facility intranet is fully or partially implemented in a cloud computing system, with the physical location of the corresponding computers being outside the premises of the chemical production facility. Thus, whether an entity is inside or outside the facility intranet depends on its communication privileges or the access authorization for the respective users rather than its location.

The process model management computer system may comprise one or more software processes or computer, all of which are outside the facility intranets. The process model management computer system may also be fully or partially implemented in a cloud computing system. It may be that the process model is only a partial method for simulating the derivative chemical production process in the sense that some physical or chemical aspects of the derivative chemical production process are deliberately simplified in the process model or omitted altogether. Each modification data may itself already present an updated process model. It may also be that the modification data only presents data which requires further computer processing, for example by additional numerical analysis, for arriving at an updated process model. Updating of the process model may comprise a modification in a causal link posited by the model. Alternatively, or in addition, updating of the process model may comprise a change in a numerical value underlying the process model or otherwise used in the process model.

According to a preferred embodiment of the method, each computing module prevents the derivative process parameters from being transferred outside the respective facility intranet. Such prevention means that, firstly, each computing module does not itself transmit the derivative process parameters to a recipient outside the respective facility intranet. Secondly, each computing module refuses requests to transfer the derivative process parameters to a recipient outside the respective facility intranet. In addition, each computing module comprises cybersecurity features, e.g. encryption and authentication mechanisms, for blocking access to the derivative process parameters from outside the respective facility intranet.

According to a further preferred embodiment of the method, each computing module prevents read access to the process model and the modification data. Such prevention of read access is irrespective of whether or not the entity requesting read access is within the respective facility intranet or without. For prevention of read access, in particular the same techniques mentioned above for blocking access may be employed. It is to be noted that despite such prevention of read access, each computing module may actively transmit in particular the respective modification data to select recipients. Preferably, such transmission is encrypted.

According to a preferred embodiment of the method, the derivative process parameters comprise respective derivative process settings and derivative production measurements. Preferably the derivative chemical products are produced through the derivative chemical production process based on the respective derivative process settings. Thus, the derivative process settings are those derivative process parameters that determine or influence the derivative chemical production process directly or indirectly. In particular, it may be that the derivative production measurements are determined from the derivative chemical production process by the respective production sensor computer system. Thus, the derivative production measurements are those derivative process parameters that result from the derivative chemical production process directly or indirectly.

According to a further preferred embodiment of the method, the derivative process parameters and in particular the derivative process settings comprise formulation data for specifying ingredients for the derivative chemical production process. Here it is preferred that the formulation data comprises specifications of proportion, weight, temperature and/or volume of respective ingredients. Such formulation data is a process parameter of especially high relevance as concerns the outcome of the derivative chemical production process.

In principle the derivative process settings may be determined in an arbitrary manner. A preferred embodiment of the method is characterized in that the derivative process settings are at least partially determined, preferably by the respective computing module, based on respective derivative product specifications regarding properties of the respective derivative chemical product in connection with the process model. In other words the respective derivative process settings are arrived at by having the respective computing module apply the respective derivative product specification on the process model. Thus, the process model and calculation based on it form the basis for determining what derivative process settings are appropriate to obtain the respective derivative product specifications in the respective derivative chemical product. In this way, a trial and error method and the associated costs are avoided. It is further preferred that that each computing module prevents the respective derivative product specifications from being transferred outside the respective facility intranet. The derivative product specifications may be as sensitive in terms of being kept confidential as the derivative process parameters.

In principle the modification data can be put a wide variety of uses. Yet according to a preferred embodiment of the method, each modification data is transmitted to an update recipient computer system outside the respective facility intranet, which update recipient computer system may in particular be the process model management computer system, from the respective computing module and the process model is updated based on the modification data. It is preferred that the process model management computer system updates the process model based on the modification data. Further it is preferred that the updated process model is transmitted to each computing module for replacing the previous process model. It may further be advantageous to repeat some or all relevant calculations or determinations performed with the process model prior to the update with the updated process model. More precise results may be expected from such repeat calculations.

According to a further preferred embodiment of the invention a respective precursor charge of precursor material for the production of each derivative chemical product is produced at a precursor production facility distant from the chemical production facility through a precursor production process, each precursor charge is transported to the respective chemical production facility and each precursor charge is used for the derivative chemical production process. The term "precursor material" is to be understood in a wide sense and therefore encompasses any material which is used and in particular consumed in the derivative chemical production process, even if it does not end up being part of the derivative chemical product. For example, the precursor material may also be a catalyst for a reaction of the derivative chemical production process. Here it is further preferred that the derivative process settings are based on respective precursor production data associated with each precursor charge. Precursor production data may be any information describing the respective precursor charge. In particular, the precursor production data may comprise precursor measurement data measured on the respective precursor charge. It may also comprise precursor production data measured during the production process of the respective precursor charge. As described in more detail below, it may also comprise data input in the production process of the respective precursor charge to determine properties of that precursor charge. Further, it may generally be that the precursor production facility is outside the above facility intranets.

In general, information regarding the production of the precursor material will have implications for the derivative chemical production process using that precursor material also. Consequently, a preferred embodiment of the method is characterized in that the derivative process parameters, in particular the derivative process settings, are at least partially determined based on the respective precursor production data in connection with the process model. Preferably, the derivative process settings are at least partially determined by the respective computing module based on the precursor production data in connection with the process model. In other words, each computing module may determine the derivative process settings by applying at least a part of the respective precursor production data to the process model. It may further be that each computing module prevents read access to the precursor production data.

The process model may also cover more processes than just the derivative chemical production process in the narrow sense. Thus, according to a preferred embodiment of the invention, the process model comprises a precursor production model for simulating the precursor production process and the precursor production data is at least partially based on respective precursor production settings for specifying the precursor production process. Thus, the precursor production process is at least partially specified by the precursor production settings.

In principle, the precursor production data may comprise the precursor production settings. The precursor production data may also comprise data derived from the precursor production settings, for example by applying an algorithm to the precursor production settings. Preferably, the precursor production data is at least partially determined in connection with the precursor production model. According to this variant, in other words the precursor production settings are applied to the precursor production model and the result of that calculation forms a basis for the precursor production data or provides the precursor production data. This precursor production data in turn forms a basis for the derivative process settings as described above. The above calculation can in principle be executed by an arbitrary computing apparatus. Here it is further preferred that the precursor production data is at least partially determined in connection with the precursor production model by the process model management computer system.

The precursor production settings may not only factor into the method at hand as an input term, but may also be a result produced by the method at hand. According to a corresponding further preferred embodiment of the invention, the precursor production settings are at least partially determined, preferably by the process model management computer system, based on respective user specifications, in particular respective derivative product specifications, in connection with the precursor production model. Thus desired properties of the derivative product may form the basis for calculating process parameters and settings of the precursor material. Preferably, on update of the process model also the precursor production settings are updated in accordance with the updated process model.

A preferred embodiment of the invention is characterized in that the precursor production data is at least partially based on respective precursor production measurements determined from the precursor production process. The precursor production data may also comprise the respective precursor production measurements, i.e. the precursor production measurements may not need to be processed further to arrive at the precursor production data. Preferably, the precursor production measurements are provided to the process model management computer system.

A further preferred embodiment of the invention is characterized in that each chemical production facility comprises respective computer controlled production devices for performing the derivative chemical production process and that the derivative process parameters, in particular the derivative process settings, are provided to the respective computer controlled production devices for controlling the derivative chemical production process. Therefore, user steps to transfer the information resulting from the process model into actual production instructions become unnecessary and the transfer can be automated. Preferably, the computer controlled production devices are within the respective facility intranet. Thus, they can be accessed more easily by the respective computing module.

According to a preferred embodiment of the invention, when the updated process model is transmitted to each computing module, that computing module updates the respective derivative process settings. Preferably, the updated derivative process settings are provided to the respective computer controlled production devices for controlling the derivative chemical production process.

A preferred embodiment of the method is characterized in that the modification data is transmitted to the process model management computer system from each computing module, that the process model is updated based on the respective modification data from each of the computing modules and that the updated process model is transmitted to each computing module for replacing the previous process model. Thus, not only does input from multiple sources benefit the process model at the process model management computer system, but the update of the process model is also fed back to the multiple sources, thereby improving the respective production processes at those facilities.

The system according to the invention is for a chemical production process and comprises a plurality of chemical production facilities for producing a respective derivative chemical product through a derivative chemical production process based on at least some derivative process parameters, which chemical production facilities each comprise a respective separate facility intranet and further comprises a respective production sensor computer system within the respective facility intranet, wherein at least some derivative process parameters are measured from the derivative chemical production process by the respective production sensor computer system. The system according to the invention further comprises a process model management computer system outside the facility intranets for recording a process model for simulating the derivative chemical production process.

The system according to the invention is characterized in that the system also comprises a respective computing module within each facility intranet, that the process model management computer system is configured to transmit the process model to each computing module and that each computing module is configured to determine respective modification data for updating the process model by a numerical analysis based on the derivative process parameters and the process model.

Preferred embodiments, features and advantages of the system according to the invention correspond to those of the method according to the invention and vice versa.

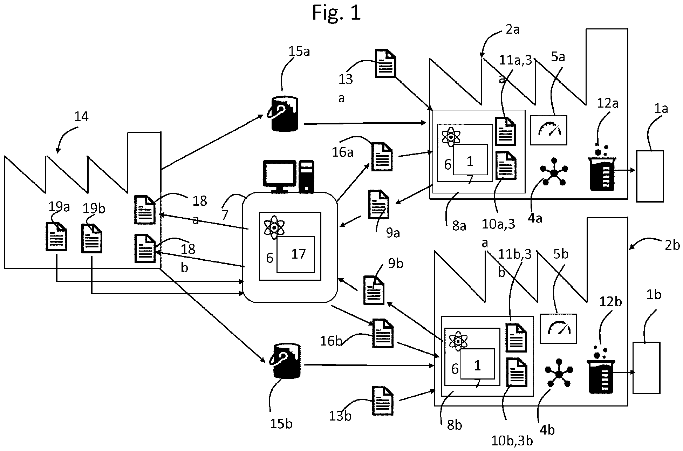

Further advantageous and preferred features are discussed in the following description with respect to the Figures. In the following it is shown in
- Fig. 1: a schematic view of an embodiment of the system according to the invention for carrying out the method according to the invention.

The system according to an embodiment of the invention shown in Fig. 1 concerns a chemical production process and in particular a derivative chemical production process for producing a derivative chemical product 1a, b, which in the present example is an isolation plate comprising polyurethane foam. In particular the derivative chemical production process involves the reaction of isocyanate and polyol resin. The described system comprises a number of chemical production facilities 2a, b, of which only two are shown, at which the derivative chemical production process is executed to produce the respective derivative chemical product 1a, b. In the present example, the respective derivative chemical product 1a, b produced at the chemical production facilities 2a, b is substantially identical in terms of their product specification.

Each chemical production facility 2a, b comprises a facility intranet 4a, b to which a respective production sensor computer system 5a, b is electronically connected. Each production sensor computer system 5a, b measures derivative production measurements 11a, b, which are measurement values derived before, during and after production from either the derivative chemical product 1a, b itself or from a respective computer controlled production device 12a, b of each chemical production facility 2a, b. The derivative chemical product 1a, b is produced by the respective computer controlled production device 12a, b based on respective derivative process settings 10a, b applied to the computer controlled production devices 12a, b. In the present example, the derivative process settings 10a, b also comprise formulation data specifying both the isocyanate and the polyol resin used in the derivative chemical production process. Further in the present example, the derivative process settings 10a, b and the derivative production measurements 11a, b form derivative process parameters 3a, b.

Within each facility intranet 4a, b, there is also a respective computing module 8a, b, which receives a process model 6 from a process model management computer system 7 outside the facility intranet 4a, b. The process model 6 is recorded in the process model management computer system 7 and presents a computational model for a numerical simulation of the aspects of interest of the derivative chemical production process. Each computing module 8a, b also receives the above-mentioned derivative process parameters 3a, b from senders within the facility intranet 4a, b, for example from the respective production sensor computer system 5a, b. Within the computing modules 8a, b, both the derivative process parameters 3a, b and the process model 6 are kept secure. In particular, the computing module 8a, b comprises encryption and other security mechanisms to prevent reading out the process model 6 from the computing module 8a, b by an external entity, in particular one from the facility intranet 4a, b. Likewise, the computing module 8a, b also prohibits transfer of the derivative process parameters 3a, b to a recipient outside the facility intranet 4a, b. This also involves preventing access by means of encryption and other suitable measures. Any such data which is no longer needed within the computing module 8a, b is securely deleted as soon as possible. In this way it is ensured that the derivative process parameters 3a, b do not leave the facility intranet 4a, b on the one hand and that the process model 6 remains restricted to the computing module 8a, b and the process model management computer system 7.

In the present example, some of the derivative process settings 10a, b are generated by the computing modules 8a, b themselves based on derivative product specifications 13a, b input by a user to the respective computing module 8a, b. These derivative product specifications 13a, b describe desired properties of the derivative chemical product 1a, b. The computing module 8a, b may then apply these derivative product specifications 13a, b to the process model 6 in order to arrive at appropriate derivative process settings 10a, b. Due to the equally sensitive nature of the derivative product specifications 13a, b, they are treated in the same way as the derivative process parameters 3a, b in terms of being kept within the facility intranet 4a, b.

The computing module 8a, b itself is fully capable of processing both the process model 6 and the derivative process parameters 3a, b. The derivative process parameters 3a, b may be derived from a plurality of runs of the derivative chemical production process and may therefore pertain to an arbitrarily large number of derivative chemical products. The computing module 8a, b is configured to run a numerical analysis, for example a regression analysis, in which the derivative process parameters 3a, b are applied to the process model 6. Because the derivative process parameters 3a, b comprise both input and output parameters of the derivative chemical production process, such parameters may be compared to a prediction generated with respect to the process model 6 and the input parameters of the derivative process parameters 3a, b. Any deviation from the predicted results may be used to update the process model 6 in a way that brings the prediction into closer alignment with the actual derivative process parameters 3a, b. Having performed this numerical analysis, the computing module 8a, b generates modification data 9a, b specifying such an update and transmits this modification data 9a, b to the process model management computer system 7 where the process model 6 is recorded and centrally managed. The modification data 9a, b may also be such that it requires further processing in order to arrive at an update of the process model 6, therefore the term may be understood in a broad sense. However, in any case the modification data 9a, b does not permit any specific conclusions about the derivative process parameters 3a, b that would compromise their confidentiality.

Within the process model management computer system 7, the actual updating of the process model 6 is performed which may involve arbitrarily complex numerical calculations and may take into account the modification data 9a, b from some or all computing modules 8a, b. The updated process model 6 is then transmitted back to all computing modules 8a, b, which then use the updated process model 6 in re-calculations of all values for which the process model 6 has formed the basis, if appropriate, for example for the derivative process settings 10a, b for production processes which have not begun yet. Also shown in Fig. 1 is a precursor production facility 14, of which there may in principle be an arbitrary number, for producing a precursor charge 15a, b of precursor material, which in the present example is polyol resin, in a precursor production process. Associated with each precursor charge 15a, b is precursor production data 16a, b, the determination of which is described in further detail below. Each precursor charge 15a, b is subsequently transported to a chemical production facility 2a, b for use in the derivative chemical production process.

The precursor production data 16a, b is then transmitted to the appropriate computing module 8a, b, where it is treated with the same restrictions on proliferation as the process model, and put to use by the computing module 8a, b by being applied to the process model 6 in numerical calculations in order to arrive at derivative process settings 10a, b. To this end, the process model 6 may comprise a precursor production model 17 specifically for simulating the precursor production process and its relationship the derivative chemical production process. In this way, the process model 6 may provide the basis for a numerical computation for determining how the derivative process settings 10a, b should be set in light of the precursor production data 16a, b associated with the precursor charge 15a, b being used and further based on the desired derivative product properties as given by the derivative product specifications 13a, b.

In the present example, some of the precursor production data 16a, b is determined by having precursor production settings 18a, b, which specify the precursor production process at the precursor production facility 14, be applied to the precursor production model 17 at the process model management computer system 7.

The precursor production settings 18a, b in turn are determined by applying user specifications, for example the above derivative product specifications 13a, b, in connection with the precursor production model 17. In the case that the derivative product specifications 13a, b are of lesser sensitivity in terms of confidentiality, such application to the precursor production model 17 may be executed in the process model management computer system 7. Alternatively, this process may be performed in a computing module 8a, b. Thus it is evident that updates of the process model 6 feed back into several stages of the total process for producing the derivative chemical product 1a, b.

Further precursor production data 16a, b is based - either directly or also by application to the precursor production model 17 - on precursor production measurements 19a, b which have been determined in particular by sensors from the precursor production process in analogy to the derivative production measurements 11a, b.

## Claims

1. Method for improving a chemical production process, wherein a plurality of derivative chemical products (1a, b) are produced through a derivative chemical production process based on at least some derivative process parameters (3a, b) at a respective chemical production facility (2a, b), which chemical production facilities (2a, b) each comprises a separate respective facility intranet (4a, b), wherein at least some respective derivative process parameters (3a, b) are measured from the derivative chemical production process by a respective production sensor computer system (5a, b) within each facility intranet (4a, b), wherein a process model (6) for simulating the derivative chemical production process is recorded in a process model management computer system (7) outside the facility intranets (4a, b), **characterized in that,** that the process model (6) is transmitted to respective computing modules (8a, b) for performing numerical analysis within each facility intranet (4a, b) from the process model management computer system (7), that the derivative process parameters (3a, b) at each chemical production facility (2a, b) are provided to the respective computing module (8a, b) and that each computing module (8a, b) determines respective modification data (9a, b) for updating the process model (6) by a numerical analysis based on the derivative process parameters (3a, b) at the respective chemical production facility (2a, b) and the process model (6).

2. Method according to claim 1, **characterized in that** each computing module (8a, b) prevents the derivative process parameters (3a, b) from being transferred outside the respective facility intranet (4a, b), preferably, that each computing module (8a, b) prevents read access to the process model (6) and the modification data (9a, b).

3. Method according to claim 1 or 2, **characterized in that** the derivative process parameters (3a, b) comprise respective derivative process settings (10a, b) and derivative production measurements (11a, b), preferably, that the derivative chemical products (1a, b) are produced through the derivative chemical production process based on the respective derivative process settings (10a, b), in particular, that the derivative production measurements (11a, b) are determined from the derivative chemical production process by the respective production sensor computer system (5a, b).

4. Method according to one of claims 1 to 3, **characterized in that** the derivative process parameters (3a, b) comprise formulation data for specifying ingredients for the derivative chemical production process, preferably, that the formulation data comprises specifications of proportion, weight, temperature and/or volume of respective ingredients.

5. Method according to claim 4, **characterized in that** the derivative process settings (10a, b) are at least partially determined, preferably by the respective computing module (8a, b), based on respective derivative product specifications (13a, b) regarding properties of the respective derivative chemical product (1a, b) in connection with the process model (6), in particular, that each computing module (8a, b) prevents the respective derivative product specifications (13a, b) from being transferred outside the respective facility intranet (4a, b).

6. Method according to one of claims 1 to 5, **characterized in that** each modification data (9a, b) is transmitted to an update recipient computer system outside the respective facility intranet, in particular the process model management computer system (7), from the respective computing module (8a, b) and that the process model (6) is updated based on the modification data (9a, b), preferably, that the updated process model (6) is transmitted to each computing module (8a, b) for replacing the previous process model (6).

7. Method according to one of claims 1 to 6, **characterized in that** a respective precursor charge (15a, b) of precursor material for the production of each derivative chemical product (1a, b) is produced at a precursor production facility (14) distant from the respective chemical production facility (1a, b) through a precursor production process, that each precursor charge (15a, b) is transported to the respective chemical production facility (2a, b) and used for the derivative chemical production process, preferably, that the derivative process settings (10a, b) are based on respective precursor production data (16a, b) associated with each precursor charge (15a, b).

8. Method according to claim 7, **characterized in that** the derivative process parameters (3a, b), in particular the derivative process settings (10a, b), are at least partially determined based on the respective precursor production data (16a, b) in connection with the process model (6), preferably, that the derivative process settings (10a, b) are at least partially determined by the respective computing module (8a, b), in particular, that each computing module (8a, b) prevents read access to the respective precursor production data (16a, b).

9. Method according to claim 7 or 8, **characterized in that** the process model (6) comprises a precursor production model (17) for simulating the precursor production process and that the precursor production data (16a, b) is at least partially based on respective precursor production settings (18a, b) for specifying the precursor production process, in particular, determined in connection with the precursor production model (17), preferably, that the precursor production data (16a, b) is at least partially determined by the process model management computer system (7).

10. Method according to claim 9, **characterized in that** the precursor production settings (18a, b) are at least partially determined, preferably by the process model management computer system (7), based on respective user specifications, in particular respective derivative product specifications (13a, b), in connection with the precursor production model (17), further preferably, that on update of the process model (6) also the precursor production settings (18a, b) are updated in accordance with the updated process model (6).

11. Method according to one of claims 7 to 10, **characterized in that** the precursor production data (16a, b) is at least partially based on respective precursor production measurements (19a, b) determined from the precursor production process, preferably, that the precursor production measurements (19a, b) are provided to the process model management computer system (7).

12. Method according to one of claims 1 to 11, **characterized in that** each chemical production (2a, b) facility comprises respective computer controlled production devices (12a, b) for performing the derivative chemical production process and that the derivative process parameters (3a, b), in particular the derivative process settings (10a, b), are provided to the respective computer controlled production devices (12a, b) for controlling the derivative chemical production process, preferably, that the computer controlled production devices (12a, b) are within the respective facility intranet (4a, b).

13. Method according to claim 12, **characterized in that** when the updated process model (6) is transmitted to each computing module, that computing module (8a, b) updates the respective derivative process settings (10a, b), preferably, that the updated derivative process settings (10a, b) are provided to the respective computer controlled production devices (12a, b) for controlling the derivative chemical production process.

14. Method according to one of claims 1 to 13, **characterized in that** the modification data (9a, b) is transmitted to the process model management computer system (7) from each computing module (8a, b), that the process model (6) is updated based on the respective modification data (9a, b) from each of the computing modules (8a, b) and that the updated process model (6) is transmitted to each computing module (8a, b) for replacing the previous process model (6).

15. System for a chemical production process comprising a plurality of chemical production facilities (2a, b) for producing a respective derivative chemical product (1a, b) through a derivative chemical production process based on at least some derivative process parameters (3a, b), which chemical production facilities (2a, b) each comprises a respective separate facility intranet (4a, b) and comprises a respective production sensor computer system (5a, b) within the respective facility intranet (4a, b), wherein at least some respective derivative process parameters (3a, b) are measured from the derivative chemical production process by the respective production sensor computer system (5a, b), wherein the system further comprises a process model management computer system (7) outside the facility intranets (4a, b) for recording a process model (6) for simulating the derivative chemical production process, **characterized in that** the system further comprises a respective computing module (8a, b) within each facility intranet (4a, b), wherein the process model management computer system (7) is configured to transmit the process model (6) to each computing module (8a, b) and wherein each computing module (8a, b) is configured to determine respective modification data (9a, b) for updating the process model (6) by a numerical analysis based on the derivative process parameters (3a, b) at the respective chemical production facility (2a, b) and the process model (6).
